# EUROPEAN PATENT SPECIFICATION

(11) **EP 3 174 535 B1**
(45) Date of publication and mention of the grant of the patent: **27.02.2019**
(21) Application number: 15754023.8
(22) Date of filing: 31.07.2015
(51) Int. Cl.: A61K 9/51, A61K 31/4196, A61K 9/20, A61K 9/48

(54) **NANOPARTICULATE FORMULATION COMPRISING A MPGES-1 INHIBITOR**
NANOTEILCHENFORMULIERUNG MIT EINEM MPGES-1-INHIBITOR
FORMULATION NANOPARTICULAIRE COMPRENANT UN INHIBITEUR DE MPGES -1

(30) Priority: 01.08.2014 IN 2472MU2014
(43) Date of publication of application: 07.06.2017
(73) Proprietor: Glenmark Pharmaceuticals S.A., 2300 La Chaux-de-Fonds (CH)
(72) Inventor: DHUPPAD, Ulhas, Nashik Maharashtra 422101 (IN); CHAUDHARI, Sunil, Nashik Maharashtra 422101 (IN); RAJURKAR, Suresh, Nashik Maharashtra 422101 (IN); JAIN, Nilesh, Nashik Maharashtra 422101 (IN); DHATRAK, Chandrakant, Nashik Maharashtra 422101 (IN); KASLIWAL, Alkesh, Nanded Maharashtra 431809 (IN)
(74) Representative: HGF Limited
(86) International application number: PCT/IB2015/055821
(87) International publication number: WO 2016/016861

(56) References cited:
- WO-A1-2011/077313
- WO-A1-2013/186692

## Description

### TECHNICAL FIELD OF THE INVENTION

The present invention relates to a nanoparticulate formulation comprising a microsomal prostaglandin E synthases-1 ("mPGES-1") inhibitor. Particularly, the present invention relates to a nanoparticulate formulation comprising an mPGES-1 inhibitor and one or more surface stabilizers; a process for preparing such formulation; and its suitability for use in treating pain and inflammation in a subject.

### BACKGROUND OF THE INVENTION

Inflammation is one of the common causes of many disorders including asthma, inflammatory bowel disease, rheumatoid arthritis, osteoarthritis, rhinitis, conjunctivitis and dermatitis. Inflammation also leads to pain. One of the major problems associated with existing treatments of inflammatory conditions is inadequate efficacy and/or the prevalence of side effects.

The enzyme cyclooxygenase (COX) converts arachidonic acid to an unstable intermediate, prostaglandin H₂ (PGH₂), which is further converted to other prostaglandins, including PGE₂, PGF_{2α}, PGD₂, prostacyclin and thromboxane A₂. Among all prostaglandin metabolites, PGE₂ is particularly known to be a strong pro-inflammatory mediator, and is also known to induce fever and pain. The conversion of PGH₂ to PGE₂ by prostaglandin E synthases (PGES) may, therefore, represent a pivotal step in the propagation of inflammatory stimuli. There are two microsomal prostaglandin E synthases (mPGES-1 and mPGES-2), and one cytosolic prostaglandin E synthase (cPGES). mPGES-1 is an inducible PGES after exposure to pro-inflammatory stimuli. mPGES-1 is induced in the periphery and CNS by inflammation, and represents therefore a target for acute and chronic inflammatory disorders. PGE₂ is a major prostanoid, produced from arachidonic acid liberated by phospholipases (PLAs), which drives the inflammatory processes. Arachidonic acid is transformed by the action of prostaglandin H synthase (PGH synthase, cycloxygenase) into PGH₂ which is a substrate for mPGES-1, the terminal enzyme transforming PGH₂ to the pro-inflammatory PGE₂.

Agents that are capable of inhibiting the action of mPGES-1, and thus reducing the formation of the specific arachidonic acid metabolite PGE₂, are beneficial in the treatment of inflammation. Blocking the formation of PGE₂ in animal models of inflammatory pain results in reduced inflammation, pain and fever response (Kojima et. al, The Journal of Immunology 2008, 180, 8361-6; Xu et. al., The Journal of Pharmacology and Experimental Therapeutics 2008, 326, 754-63).

International Publication Nos. WO 2006/063466, WO 2007/059610, WO 2010/034796, WO 2010/100249, WO 2012/055995, WO 2012/110860 and WO 2013/038308 disclose numerous heterocyclic compounds which are stated to be inhibitors of the microsomal prostaglandin E synthase-1 (mPGES-1) enzyme.

U.S. Patent Nos. 5145684 and 7998507 and PCT Application Publication No. WO2003//049718 disclose nanoparticulate compositions.

There is a need for new, improved formulations of mPGES-1 inhibitors and methods of making and using such formulations.

### SUMMARY OF THE INVENTION

The present invention relates to a nanoparticulate formulation comprising an mPGES-1 inhibitor, for example a poorly soluble mPGES-1 inhibitor such as the compound *N*-(4-chloro-3-(5-oxo-1-(4-(trifluoromethyl)phenyl)-4,5-dihydro-1*H*-1,2,4-triazol-3-yl)benzyl)pivalamide or its pharmaceutically acceptable salt, solvates, hydrates or other derivative including esters and prodrug. The nanoparticulate formulation provides enhanced dissolution of the mPGES-1 inhibitor. Furthermore, the nanoparticles of the present invention are stable (e.g., with respect to particle size distribution, dissolution profile, and drug content over time) and provide a desirable dissolution profile.

In one embodiment, the nanoparticulate formulation comprises the compound *N*-(4-chloro-3-(5-oxo-1-(4-(trifluoromethyl)phenyl)-4,5-dihydro-1*H*-1,2,4-triazol-3-yl)benzyl)pivalamide (compound I) or its pharmaceutically acceptable salt and one or more surface stabilizers.

The nanoparticles preferably comprise the mPGES-1 inhibitor and one or more surface stabilizers.

In one of the embodiment nanoparticulate formulation comprises a compound *N*-(4-chloro-3-(5-oxo-1-(4-(trifluoromethyl)phenyl)-4,5-dihydro-1*H*-1,2,4-triazol-3-yl)benzyl)pivalamide (compound I) or its pharmaceutically acceptable salt and one or more surface stabilizer selected from the group consisting of polymers (also referred to as a polymer stabilizer or polymeric stabilizer) and surfactants. The compound I acts as an mPGES-1 inhibitor in the formulations and pharmaceutical compositions described herein.

In another embodiment, the nanoparticulate formulation comprises compound I or a pharmaceutically acceptable salt thereof wherein compound I or pharmaceutically acceptable salts thereof, has an effective average particle size in the range from about 20 nm to about 1000 nm. The formulation may comprise a therapeutically effective amount of compound I or its pharmaceutically acceptable salt, for example, an amount effective to inhibit mPGES-1 in a subject. The nanoparticulate formulation may further comprise one or more pharmaceutically acceptable excipients.

In an embodiment, the nanoparticulate particles may exist in a crystalline phase, an amorphous phase, a semi-crystalline phase, a semi amorphous phase, or a mixture thereof.

In one embodiment, the nanoparticulate formulation comprises from about 2% to about 15% by weight of an mPGES-1 inhibitor (such as compound I or a pharmaceutically acceptable salt thereof), such as from about 5 to about 10% by weight of an mPGES-1 inhibitor, based upon 100% total weight of the formulation.

In another embodiment, the nanoparticulate formulation comprises from about 15% to about 80% by weight of an mPGES-1 inhibitor (such as compound I or a pharmaceutically acceptable salt thereof) based upon 100% total weight of the formulation.

A nanoparticulate formulation comprising a compound *N*-(4-chloro-3-(5-oxo-1-(4-(trifluoromethyl)phenyl)-4,5-dihydro-1*H*-1,2,4-triazol-3-yl)benzyl)pivalamide (compound I) or its pharmaceutically acceptable salt and one or more surface stabilizers selected from a group consisting of a polymer and a surfactant.

In one embodiment, the surface stabilizer may be a polymer selected from one or more from polyvinyl pyrrolidone, copovidone, hydroxypropyl cellulose, hydroxyethyl cellulose, hydroxypropylmethyl cellulose, polyethylene glycol, natural gums, cellulose derivatives and combinations thereof.

In another embodiment, the weight ratio of the mPGES-1 inhibitor (such as compound I or a pharmaceutically acceptable salt thereof) to the polymer stabilizer ranges from about 1:0.01 to about 1:100, or more preferable from about 1:0.1 to about 1:50.

In another embodiment, the nanoparticulate formulation comprises an mPGES1 inhibitor (such as compound 1 or its pharmaceutically acceptable salt) and one or more surface stabilizers wherein the surface stabilizer is a surfactant selected from poloxamer, polyoxyethylene sorbitan esters, polyethoxylated castor oil, glycerol monostearate, phospholipids, benzalkonium chloride, triethanolamine, sodium lauryl sulfate, docusate sodium, vitamin E TPGS, soya lecithin, or combinations thereof.

The nanoparticulate formulation may have a weight ratio of the mPGES-1 inhibitor (such as compound I or its pharmaceutically acceptable salt) to the surfactant ranging from about 1:0.01 to about 1:100 or from about 1:0.1 to about 1:50.

Another embodiment relates to nanoparticulate formulation comprising a compound I or its pharmaceutically acceptable salt, a polymer and a surfactant, wherein the formulation has an effective average particle size in the range from about 20 nm to about 1000 nm.

In another embodiment, the formulation has an effective average particle size in the range from about 50 nm to about 600 nm, more preferably from about 70 nm to 500 nm, more preferably from about 80 nm to 400 nm.

In one embodiment, the nanoparticles have a D₁₀ value in the range from about 10 nm to about 300 nm, or preferably from about 20 nm to about 200 nm. In another embodiment, the nanoparticles have a D₈₀ value in the range from about 100 nm to about 1000 nm, or preferably from about 200 nm to about 800 nm.

In yet another embodiment, the effective average particle size is in the range from about 70 nm to about 500 nm or from about 80 nm to about 400 nm. In one aspect of this embodiment, the D₁₀ value is in the range from about 50 nm to about 200 nm. In another aspect the D₈₀ value is in the range from about 300 nm to about 800 nm.

In one embodiment, the nanoparticulate formulation comprises an mPGES1 inhibitor (compound 1 or a pharmaceutically acceptable salt thereof) and one or more surface stabilizers wherein the surface stabilizer is selected from polymer such as polyvinyl pyrrolidone, copovidone, hydroxypropyl cellulose, hydroxyethyl cellulose, hydroxypropylmethyl cellulose, polyethylene glycol, natural gums, cellulose derivatives and combinations thereof. The weight ratio of the compound I or its pharmaceutically acceptable salt to the polymer may range from about 1:0.01 to 1:100 or about 1:0.1 to about 1:50.

In another embodiment, the said nanoparticulate formulation comprises an mPGES1 inhibitor (compound I or pharmaceutically acceptable salt thereof) and one or more surface stabilizers wherein the stabilizer is selected from surfactants such as poloxamer, polyoxyethylene sorbitan esters, polyethoxylated castor oil, glycerol monostearate, phospholipids, benzalkonium chloride, triethanolamine, sodium lauryl sulfate, docusate sodium, vitamin E TPGS, soya lecithin, and combinations thereof. The weight ratio of compound I or its pharmaceutically acceptable salt to the surfactant may range from about 1:0.01 to about 1:100 or from about 1:0.1 to about 1:50.

Yet another embodiment is a nanoparticulate formulation comprising i) an mPGES-1 inhibitor (such as compound I or a pharmaceutically acceptable salt thereof), ii) mannitol, iii) sodium lauryl sulphate, iv) hydroxy propyl methyl cellulose, v) poloxamer or vitamin ETPGS, wherein the formulation has an effective average particle size in the range from about 70 nm to about 500 nm, more preferably from 80 nm to 400 nm.

Another embodiment is a pharmaceutical composition comprising the nanoparticulate formulation described herein. The pharmaceutical formulation can be in the form of various dosage forms including, but not limited to, a dispersion, gel, aerosol, ointment, cream, lotion, paste, spray, film, patch, tablet, capsules, powder, granules, dry syrup, syrup or parenteral preparation such as preparation for intravenous, intra-arterial, intramuscular, intra-articular, or subcutaneous injection.

In a preferred embodiment, the pharmaceutical composition is present in the form of a dispersion, liquid solution, suspension, semi-solid preparation, granules, powders, tablet or capsules.

In one embodiment, the present invention relates to a pharmaceutical composition comprising a nanoparticulate formulation of the invention and one or more pharmaceutically acceptable excipients.

In an embodiment, the present invention also relates to a pharmaceutical composition comprising a nanoparticulate formulation comprising particles of an mPGES-1 inhibitor (such as compound I or a pharmaceutically acceptable salt thereof), one or more surface stabilizers and one or more pharmaceutically acceptable excipients. The nanoparticles have an effective average particle size in the range from about 20 nm to about 1000 nm.

The nanoparticulate formulation can be administered by an appropriate route which includes, but is not limited to, the oral, pulmonary, rectal, ophthalmic, parenteral, intravaginal, local, buccal, nasal or topical route. Preferably, the nanoparticulate formulation is suitable for oral administration.

In one embodiment, the pharmaceutical composition described herein is an immediate release composition suitable for oral administration.

In another embodiment, the pharmaceutical composition is an extended release or a delayed release composition suitable for oral administration.

Yet another embodiment is a process for the preparation of a nanoparticulate formulation comprising an mPGES-1 inhibitor (such as compound I or a pharmaceutically acceptable salt thereof) and one or more surface stabilizers. The process may include (a) reducing the size of particles in an aqueous suspension, where the particles comprise an mPGES-1 inhibitor and one or more surface stabilizer (e.g., to an average particle size below 1000 nm), and (b) optionally spray drying the suspension. The particles in step (a) may be reduced by any method known in the art, including with a bead mill or high pressure wet milling. In one embodiment, the process comprises the steps of:
a) mixing an mPGES-1 inhibitor with one or more surface stabilizer, water and optionally other excipients to form an aqueous suspension;
b) reducing the particle size of the aqueous suspension (for example with a bead mill or high pressure wet milling) and
c) spray drying of aqueous suspension.

Yet another embodiment is a process for the preparation of a nanoparticulate formulation comprising an mPGES-1 inhibitor (such as compound I or a pharmaceutically acceptable salt thereof) and one or more surface stabilizers. The process comprises the steps of:
a) reducing the particle size of the compound I or its pharmaceutically acceptable salt for example with a bead mill or high pressure wet milling;
b) mixing compound I or its pharmaceutically acceptable salt with the surface stabilizer, water and optionally other excipients to form an aqueous suspension and
c) spray drying of an aqueous suspension.

Yet another embodiment is a process for preparation of a nanoparticulate formulation comprising the mPGES-1 inhibitor (such as compound I or a pharmaceutically acceptable salt thereof) and one or more surface stabilizer which is a mixture of a polymer (i.e., a polymeric stabilizer) and a surfactant. The process comprises the steps of:
1. dissolving polymeric stabilizer (such as copovidone and sodium lauryl sulphate) in water (e.g., purified water),
2. dissolving surfactant (such as poloxamer) in water (e.g., purified water) and adding the same to the solution of step 1,
3. adding the mPGES-1 inhibitor to the solution of step 2 to form a suspension (uniform suspension),
4. milling the suspension of step 3 to get desired particle size,
5. sifting the milled suspension of step 4,
6. spray drying the milled suspension of step 5 to obtain granules, and
7. filling the granules of step 6 in a pouch (e.g., a triple aluminum laminate pouch) or optionally filling in capsules or optionally compressing into tablets.

The present invention also relates to a nanoparticle formulation for the treatment of an inflammation and/or pain in a subject, comprising compound I or its pharmaceutically acceptable salt and one or more surface stabilizer, wherein the formulation has an effective average particle size in the range from about 20 nm to about 1000 nm.

In one embodiment, the present invention relates a nanoparticle formulation for the treatment of an inflammation and/or pain or a disease or condition associated with pain and/or inflammation in a subject, comprising the compound I or a pharmaceutically acceptable salt thereof and a surface stabilizer; wherein the nanoparticles have an effective average particle size in the range from about 20 nm to 1000 nm, preferably from about 30 nm to about 800 nm, preferably from about 50nm to about 600nm, more preferably from about 70 nm to about 500 nm, more preferably from about 80 nm to about 400 nm.

In a further embodiment, the nanoparticulate formulation can be administered to the subject in need thereof once daily, twice daily, thrice daily or four times a day.

In yet another embodiment, the nanoparticulate formulation can be administered to a subject in need thereof at a dose range of about 10mg to about 500mg of compound I or its pharmaceutically acceptable salt.

### DETAILED DESCRIPTION OF THE INVENTION

### Definitions

The term "active ingredient" (used interchangeably with "active" or "active substance" or "drug") as used herein refers to an mPGES -1 inhibitor. Preferably, the mPGES -1 inhibitor is *N*-(4-chloro-3-(5-oxo-1-(4-(trifluoromethyl)phenyl)-4,5-dihydro-1*H*-1,2,4-triazol-3-yl)benzyl)pivalamide (hereinafter, "compound I") having structural formula: 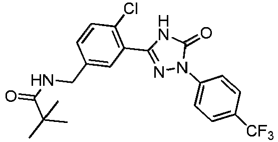 or its pharmaceutically acceptable salt, solvate, hydrate or other derivatives including esters and prodrug.

By "salt" or "pharmaceutically acceptable salt", it is meant those salts which are, within the scope of sound medical judgment, suitable for use in contact with the tissues of humans and lower animals without undue toxicity, irritation, and allergic response, commensurate with reasonable benefit to risk ratio, and effective for their intended use. Representative acid additions salts include hydrochloride, hydrobromide, sulphate, bisulphate, acetate, oxalate, valerate, oleate, palmitate, stearate, laurate, borate, benzoate, lactate, phosphate, tosylate, mesylate, citrate, maleate, fumarate, succinate, tartrate, ascorbate, glucoheptonate, lactobionate, and lauryl sulphate salts. Representative alkali or alkaline earth metal salts include sodium, calcium, potassium and magnesium salts.

The term "surface stabilizer" as used herein includes agents which associate with the surface of particles of the mPGES -1 inhibitor, but do not chemically bond to or interact with it. Without being bound by any particular theory, it is believed that the surface stabilizer provides steric and/or ionic barriers to prevent agglomeration of the particles.

The term "nanoparticulate formulation" as used herein refers to a pharmaceutical dispersion wherein drug particles are dispersed in a solvent and have an effective average particle size of less than about 1000 nm.

As used herein, the term "average particle size" (or synonymously, "mean particle size") refers to the distribution of particles, wherein about 50 volume percent of all the particles measured have a size less than the defined average particle size value. This can be identified by the term "D₅₀" or "d (0.5)".

As used herein, the term "D₁₀" refers to the distribution of particles wherein about 10 volume percent of all the particles measured have a size less than the defined particle size value. This can be identified by the term "d (0.1)" as well. Similarly, as used herein, the term "D₈₀" refers to the distribution of particles wherein about 80 volume percent of all the particles measured have a size less than the defined particle size value. This can be identified by the term "d (0.8)" as well. On similar lines, as used herein, the term "D₉₀" refers to the distribution of particles wherein about 90 volume percent of all the particles measured have a size less than the defined particle size value. This can be identified by the term "d (0.9)" as well.

The particle size can be measured using various techniques such as laser diffraction, photon correlation spectroscopy (PCS) and Coulter's principle. When PCS is used as the method of determining particle size, the average particle size is the Z-average particle diameter known to those skilled in the art. Typically, instruments such as a ZETASIZER® 3000 HS (Malvern® Instruments Ltd., Malvern, United Kingdom), NICOMP 388™ ZLS system (PSS-Nicomp Particle Sizing Systems, Santa Barbara, CA, USA), or Coulter Counter are used to determine the average particle size. Preferably, a Mastersizer 2000 (Malvern® Instruments Ltd., Malvern, United Kingdom) is used to determine the particle size of the particles.

By "an effective average particle size in the range from about 20 nm to about 1000 nm" it is meant that at least 50% of the total particles of compound I or its salt have a particle size in the range from about 20 nm to about 1000 nm when measured by the techniques mentioned herein. It is preferred that at least about 80% or at least about 90% of the particles have a particle size less than the effective average particle size, e.g., 1000 nm.

By "an effective average particle size in the range from about 30 nm to about 800 nm" it is meant that at least 50% of the total particles of compound I or its salt have a particle size in the range from about 30 nm to about 800 nm when measured by the techniques mentioned herein.

By "an effective average particle size in the range from about 50 nm to about 600 nm" it is meant that at least 50% of the total particles of compound I or its salt have a particle size in the range from about 50 nm to about 600 nm when measured by the techniques mentioned herein.

By "an effective average particle size in the range from about 70 nm to about 500 nm" it is meant that at least 50% of the total particles of compound I or its salt have a particle size in the range from about 70 nm to about 500 nm when measured by the techniques mentioned herein.

By "an effective average particle size in the range from about 80 nm to about 400 nm" it is meant that at least 50% of the total particles of compound I or its salt have a particle size in the range from about 80 nm to about 400 nm when measured by the techniques mentioned herein.

By "pharmaceutically acceptable excipient" it is meant any of the components of a formulation or pharmaceutical composition other than the active ingredient, and which are approved by regulatory authorities or are generally regarded as safe for human or animal use.

The term "treating" or "treatment" as used herein includes the prophylaxis, mitigation, prevention, amelioration, or suppression of a disorder modulated by the mPGES -1 inhibitor in a subject.

The term "effective amount" or "therapeutically effective amount" when used in conjunction with an mPGES-1 inhibitor denotes an amount of an active ingredient that, when administered to a subject for treating a state, disorder or condition, produces an intended therapeutic benefit in a subject.

The term "subject" includes mammals such as humans and other animals, such as domestic animals (e.g., household pets including cats and dogs) and non-domestic animals (such as wildlife). Preferably, the subject is a human.

"Pain" a complex constellation of unpleasant sensory, emotional and cognitive experiences provoked by real or perceived tissue damage and manifested by certain autonomic, psychological and behavioral reactions and is a disease of epidemic proportions. From a neurobiological perspective, pain is believed to be of three different aspects: first, pain that is an early warning physiological protective system, essential to detect and minimize contact with damaging or noxious stimuli and is called 'nociceptive pain'; second, pain is adaptive and protective, by heightening sensory sensitivity after unavoidable tissue damage, which is mainly caused by activation of the immune system by tissue injury or infection and is normally called 'inflammatory pain'; and the third type of pain which is not protective, but maladaptive resulting from abnormal functioning of the nervous system and generally called as 'pathological pain'. This pathological pain is not a symptom of some disorder but rather a disease state of the nervous system and can occur after damage to the nervous system (neuropathic pain) or a situation where there is no such damage or inflammation (dysfunctional pain - like fibromyalgia, irritable bowel syndrome, temporomandibular joint disease, interstitial cystitis and other syndromes where there is substantial pain but no noxious stimulants and minimal/no peripheral inflammatory pathology).

Pain can also have different qualities and temporal features depending on the modality and locality of the stimulus, respectively - firstly pain can be described as lancinating, stabbing or pricking; and secondly more pervasive including burning, throbbing, cramping, aching and sickening. It is believed to be one of the leading causes of joint movement limitations and disability.

### The mPGES-1 inhibitor

Suitable mPGES-1 inhibitors include, but are not limited to, those disclosed in coassigned International Publication No. WO 2013/186692 ("the '692 application"). These mPGES-1 inhibitors are useful for the treatment of pain and inflammation in a variety of diseases and conditions. One preferred mPGES-1 inhibitor disclosed in the '692 application is *N*-(4-chloro-3-(5-oxo-1-(4-(trifluoromethyl)phenyl)-4,5-dihydro-1*H*-1,2,4-triazol-3-yl)benzyl)pivalamide (hereinafter, "compound I") having the structural formula: 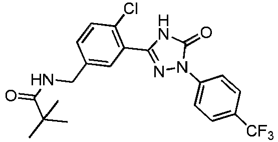 or its pharmaceutically acceptable salt, solvates and hydrates and other derivatives including esters and prodrugs.

### Surface Stabilizer

The surface stabilizer may be one or more polymers, one or more surfactants, or a combination thereof. Suitable polymers include, but are not limited to, cellulose derivatives, such as hydroxypropyl methyl cellulose(hypromellose), hydroxypropyl cellulose, methyl cellulose, carboxymethyl cellulose sodium or calcium salt, hydroxyl ethyl cellulose, polyvinyl pyrrolidone, copovidone, carbopols, copolymers of polyvinyl pyrrolidone, polyoxyethylene alkyl ether, polyethylene glycol, co-block polymers of ethylene oxide and propylene oxide (poloxamer®, Pluronic®), poly methacrylate derivatives, polyvinyl alcohol, polyvinyl alcohol derivatives and polyethylene glycol derivatives, such as macrogol glycerol stearate, natural gums such as xanthan gum, locust bean gum, alginic acid, carrageenan, and sodium alginate. Preferred polymers include, but are not limited to, polyvinyl pyrrolidone, copovidone, hydroxypropyl cellulose, hydroxyethyl cellulose, hydroxypropylmethyl cellulose, polyethylene glycol, magnesium aluminum silicate, cellulose derivatives and natural gums.

Suitable surfactants include, but are not limited to, poloxamer, polyoxyethylene sorbitan esters (such as polysorbate or Tween® available from Sigma-Aldrich of St. Louis, MO), polyethoxylated castor oil (such as Cremophor® available from BASF of Florham Park, N.J.), methyl glucose sesquistearate, PEG-20 methyl glucoside sesquistearate, caprylocaproyl macrogol-8 glycerides, lauroyl macrogol-32- glycerides, Steareth-21, soluplus, polyethylene glycol 20 sorbitan monostearate, polyethylene glycol 60 sorbitan monostearate, polyethylene glycol 80 sorbitan monostearate, Steareth-20, Ceteth-20, PEG-100 stearate, sodium stearoyl sarcosinate, hydrogenated lecithin, sodium cocoylglyceryl sulfate, sodium stearyl sulfate, sodium stearoyl lactylate, PEG-20 glyceryl monostearate, sucrose monostearate, sucrose polystearates, polyglyceryl 10 stearate, polyglyceryl 10 myristate, steareth 10, DEA oleth 3 phosphate, DEA oleth 10 phosphate, PPG-5 Ceteth 10 phosphate sodium salt, PPG-5 Ceteth 10 phosphate potassium salt, steareth-2, PEG-5 soya sterol oil, PEG- 10 soya sterol oil, diethanolamine cetyl phosphate, sorbitan monostearate, diethylenglycol monostearate, glyceryl monostearate, sodium stearyl sulfate, benzalkonium chloride, docusate sodium, triethanolamine, and phospholipids. Preferred surfactants include, but are not limited to, polyoxyethylene sorbitan esters (such as polysorbate or Tween®), polyethoxylated castor oil (such as cremophor®), glycerol monostearate, phospholipids, benzalkonium chloride, triethanolamine, sodium lauryl sulfate, docusate sodium, Vitamin E TPGS, and soya lecithin. In one embodiment, the surfactant is selected from poloxamer, polyoxyethylene sorbitan esters (such as polysorbate or Tween®), polyethoxylated castor oil (such as Cremophor®), glycerol monostearate, phospholipids, benzalkonium chloride, triethanolamine, sodium lauryl sulfate, docusate sodium, vitamin E TPGS, and soya lecithin.

### Nanoparticulate Formulations

One embodiment is a nanoparticulate formulation comprising an mPGES-1 inhibitor, such as the compound *N*-(4-chloro-3-(5-oxo-1-(4-(trifluoromethyl)phenyl)-4,5-dihydro-1*H-*1,2,4-triazol-3-yl)benzyl)pivalamide (compound I) or its pharmaceutically acceptable salt and one or more surface stabilizers.

In an embodiment, the said nanoparticulate formulation may further comprise one or more pharmaceutically acceptable excipients.

The present invention relates to a nanoparticulate formulation comprising a compound *N*-(4-chloro-3-(5-oxo-1-(4-(trifluoromethyl)phenyl)-4,5-dihydro-1*H*-1,2,4-triazol-3-yl)benzyl)pivalamide (compound 1) or its pharmaceutically acceptable salt and one or more surface stabilizers selected from the group consisting of polymers or surfactants.

In another embodiment, the nanoparticulate formulation comprising the compound I or a pharmaceutically acceptable salt thereof wherein compound I having an effective average particle size in the range from about 20 nm to about 1000 nm. The nanoparticulate formulation may further comprise one or more pharmaceutically acceptable excipients.

In one embodiment, the nanoparticulate formulation has an effective average particle size in the range from about 30 nm to about 800 nm, preferably from about 50 nm to about 600 nm, more preferably from about 80 nm to about 400 nm.

In one embodiment, the nanoparticulate formulation comprise from about 2 to about 15% by weight of an mPGES-1 inhibitor (such as compound I or a pharmaceutically acceptable salt thereof), such as from about 5 to about 10% by weight of an mPGES-1 inhibitor, based upon 100% total weight of the formulation.

In another embodiment, the said nanoparticulate formulation comprise from about 15 to about 80% by weight of an mPGES-1 inhibitor (such as compound I or a pharmaceutically acceptable salt thereof) based upon 100% total weight of the formulation.

In a preferred embodiment, the present invention provides a nanoparticulate formulation comprising compound I or a pharmaceutically salt thereof and a surface stabilizer selected from a polymer, a surfactant, and/or combination thereof.

The formulation may have an effective average particle size in the range from about 30 nm to about 800 nm, or preferably from about 50 nm to about 600 nm, more preferably from about 80 nm to about 400 nm.

Another embodiment is a nanoparticulate formulation comprising particles of compound I or a pharmaceutically salt thereof and a surface stabilizer selected from polyvinyl pyrrolidone, copovidone, hydroxypropyl cellulose, hydroxyethyl cellulose, hydroxypropylmethyl cellulose, polyethylene glycol, natural gums, cellulose derivatives and combinations thereof., where the particles have an effective average particle size in the range from about 20 nm to about 1000 nm, from about 30 nm to about 800 nm, or from about 50 nm to about 600 nm.

In one embodiment, surface stabilizer comprises copovidone, poloxamer, sodium lauryl sulfate, and polyethylene glycol, and any combination of any of the foregoing. The particles may also include a diluent, such as mannitol. In one preferred embodiment, the particles have an effective average particle size in the range from about 70 nm to about 500 nm or from about 80 nm to about 400 nm.

In one embodiment, the surface stabilizer is selected from one or more polymers selected from polyvinyl pyrrolidone, copovidone, hydroxypropyl cellulose, hydroxyethyl cellulose, hydroxypropylmethyl cellulose, polyethylene glycol, natural gums, cellulose derivatives and combinations thereof.

In another embodiment, the weight ratio of the mPGES-1 inhibitor (such as compound I or a pharmaceutically acceptable salt thereof) to the polymer stabilizer ranges from about 1:0.01 to about 1:100, or more preferable from about 1:0.1 to about 1:50.

In another embodiment, the nanoparticulate formulation comprises an mPGES1 inhibitor (such as compound I or its pharmaceutically acceptable salt) and a surface stabilizer which is a surfactant selected from poloxamer, polyoxyethylene sorbitan esters, polyethoxylated castor oil, glycerol monostearate, phospholipids, benzalkonium chloride, triethanolamine, sodium lauryl sulfate, docusate sodium, vitamin E TPGS, soya lecithin, or combinations thereof.

In further embodiment, the nanoparticulate formulation may have a weight ratio of the mPGES-1 inhibitor (such as compound I or its pharmaceutically acceptable salt) to the surfactant ranging from about 1:0.01 to about 1:100 or from about 1:0.1 to about 1:50.

In another embodiment, the nanoparticles have a D₁₀ value in the range from about 1 nm to about 500 nm, or preferably from about 5 nm to about 200 nm. In another aspect of this embodiment, the nanoparticles have a D₈₀ value in the range from about 100 nm to about 1000 nm, or preferably from about 200 nm to about 800 nm.

In yet another embodiment, the effective average particle size of the nanoparticles is in the range from about 70 nm to about 500 nm or from about 80 nm to about 400 nm. In one aspect of this embodiment, the D₁₀ value is in the range from about 5 nm to about 200 nm. In another aspect the D₈₀ value is in the range from about 300 nm to about 800 nm.

All combinations of the particle size ranges are contemplated to be within the scope of this invention. For example, the nanoparticles may have a D₁₀ value of from about 1 nm to about 500 nm as well as a D₈₀ value of from about 200 to about 800 nm.

In another embodiment, the surfactant is selected from poloxamer, polyoxyethylene sorbitan esters, polyethoxylated castor oil, glycerol monostearate, phospholipids, benzalkonium chloride, triethanolamine, sodium lauryl sulfate, docusate sodium, Vitamin E TPGS, soya lecithin, and any combination thereof.

In another embodiment, the present invention relates to a nanoparticulate formulation comprising an mPGES-1 inhibitor (such as Compound I or a pharmaceutically acceptable salt thereof), mannitol, sodium lauryl sulphate, Hydroxy propyl methyl cellulose, poloxamer or vitamin ETPGS.

Yet another embodiment is a nanoparticulate formulation comprising i) an mPGES-1 inhibitor (such as compound I or a pharmaceutically acceptable salt thereof), ii) mannitol, iii) sodium lauryl sulphate, iv) hydroxy propyl methyl cellulose, and poloxamer or vitamin ETPGS, wherein the formulation has an effective average particle size in the range from about 70 nm to about 500 nm, more preferably from 80nm to 400nm.

The nanoparticles may further include one or more pharmaceutically acceptable excipients, such as a diluent. Non-limiting examples of diluents include one or more of microcrystalline cellulose, silicified microcrystalline cellulose (e.g., Prosolv®), microfine cellulose, lactose, starch, pregelatinized starch, mannitol, sorbitol, dextrates, dextrin, maltodextrin, dextrose, calcium carbonate, calcium sulfate, dibasic calcium phosphate dihydrate, tribasic calcium phosphate, magnesium carbonate, magnesium oxide, and combinations thereof. Other examples of diluents include (1) cores or beads comprising insoluble inert materials such as glass particles / beads or silicon dioxide, calcium phosphate dihydrate, dicalcium phosphate, calcium sulfate dihydrate, or cellulose derivatives; (2) soluble cores such as sugar spheres of sugars such as dextrose, mannitol, sorbitol, or sucrose; (3) insoluble inert plastic materials such as spherical or nearly spherical core beads of polyvinyl chloride, polystyrene or any other pharmaceutically acceptable insoluble synthetic polymeric material, 4) acacia, guar gum, alginic acid, dextrin, maltodextrin, methylcellulose, ethyl cellulose, hydroxyethyl cellulose, hydroxypropyl cellulose (e.g., Klucel®), low substituted hydroxypropyl cellulose, hydroxypropyl methylcellulose (e.g., Methocel®), carboxymethyl cellulose sodium, povidone (various grades of Kollidon®, Plasdone®), carboxymethyl cellulose calcium, croscarmellose sodium, (e.g., Ac-Di-Sol®, Primellose®), crospovidone (e.g., Kollidon®, Polyplasdone®), povidone K-30, polacrilin potassium, sodium starch glycolate (e.g., Primogel, Explotab®), and combinations thereof.

### Pharmaceutical Compositions

The present invention relates to the nanoparticulate formulation which can be administered by an appropriate route which includes, but is not limited to, the oral, pulmonary, rectal, ophthalmic, parenteral, intravaginal, local, buccal, nasal or topical route. Preferably, the nanoparticulate formulation is suitable for oral administration.

The nanoparticulate formulation can be converted or incorporated into a suitable pharmaceutical composition which includes, but is not limited to, dispersion, gel, aerosol, ointment, cream, lotion, paste, spray, film, patch, tablets, capsules, powder, granules, dry syrup, syrup and parenteral preparations such as intravenous, intra-arterial, intramuscular, intra-articular, and subcutaneous injections.

In a preferred embodiment, the nanoparticulate formulation is in the form of a dispersion, liquid suspension, semi-solid suspension, powder, granules, tablets or capsules.

In one embodiment, the pharmaceutical composition is an immediate release composition suitable for oral administration.

In another embodiment, the pharmaceutical composition is an extended release or a delayed release composition suitable for oral administration. The nanoparticulate formulation of the present invention can be administered as such, or alternately, it can be further converted into a suitable pharmaceutical composition such as solid, liquid or semi-solid preparation for ease of administration. The pharmaceutical composition may be prepared by conventional methods known in the art.

In one embodiment, the present invention relates to a pharmaceutical composition comprising the nanoparticulate formulation of the invention and one or more pharmaceutically acceptable excipients.

Suitable pharmaceutically acceptable excipients include, but are not limited to one or more of diluents, glidants and lubricants, preservatives, buffering agents, chelating agents, polymers, opacifiers, colorants, gelling agents and viscosifying agents, antioxidants, disintegrants, solvents, co-solvents, and combinations thereof.

Non-limiting examples of glidants and lubricants include one or more of stearic acid, magnesium stearate, talc, colloidal silicon dioxide, and sodium stearyl fumarate.

Non-limiting examples of preservatives include one or more of phenoxyethanol, parabens such as methyl paraben and propyl paraben and their sodium salts, propylene glycols, sorbates, urea derivatives such as diazolindinyl urea, and mixtures thereof. Non-limiting examples of buffering agents include sodium hydroxide, potassium hydroxide, ammonium hydroxide and mixtures thereof. Non-limiting examples of chelating agents include ethylene diamine tetraacetic acid ("EDTA"), and disodium edetate and EDTA derivatives.

Non-limiting examples of polymers include one or more of gum arabic, sodium based lignosulfonate, methyl methacrylate, methacrylate copolymers, isobutyl methacrylate, and ethylene glycol dimethacrylate.

Non-limiting examples of gelling agents and viscosifying agents include one or more of carbomers (carbopol), modified cellulose derivatives, naturally-occurring, synthetic or semi-synthetic gums such as xanthan gum, acacia and tragacanth, sodium alginate, gelatin, modified starches, cellulosic polymers such as hydroxypropyl cellulose, hydroxyethyl cellulose, hydroxypropyl methylcellulose, hydroxypropyl methylcellulose phthalate, and methyl cellulose, co-polymers such as those formed between maleic anhydride and methyl vinyl ether, colloidal silica, methacrylate derivatives, polyethylene oxides, polyoxyethylene-polyoxypropylene copolymers, and polyvinyl alcohol.

Non-limiting examples of co-solvents include one or more of propylene glycol, polyol esters of fatty acids, trialkyl citrate esters, propylene carbonate, dimethylisosorbide, ethyl lactate, N-methylpyrrolidones, transcutol, glycofurol, decaglycerol mono-, dioleate (Caprol PGE-860), triglycerol monooleate (Caprol 3GO), polyglycerol oleate (Caprol MPGO), mixed diesters of Caprylic/Capric acid and propylene glycol (Captex 200), glyceryl mono- and dicaprate (Capmul MCM), isostearyl isostearate, oleic acid, peppermint oil, oleic acid, soybean oil, safflower oil, corn oil, olive oil, cottonseed oil, arachis oil, sunflower seed oil, palm oil, rapeseed oil, ethyl oleate, glyceryl monooleate, and vitamin E TPGS.

Non-limiting examples of solvents include one or more of water; tetrahydrofuran; propylene glycol; liquid petrolatum; ether; petroleum ether; alcohols, e.g., methanol, ethanol, isopropyl alcohol and higher alcohols; alkanes, e.g., pentane, hexane and heptane; ketones, e.g., acetone and methyl ethyl ketone; chlorinated hydrocarbons, e.g., chloroform, carbon tetrachloride, methylene chloride and ethylene dichloride; acetates, e.g., ethyl acetate; lipids, e.g., isopropyl myristate, diisopropyl adipate and mineral oil.

The nanoparticulate formulations and pharmaceutical compositions are stable (e.g., with respect to particle size distribution, dissolution profile, and drug content over time) and provide a desirable dissolution profile. For example, in one embodiment, the nanoparticulate formulation or pharmaceutical composition exhibits less than a 4, 5, or 10% variation in the amount of drug dissolved in 60 minutes when tested initially and after 3 or 6 months of storage under standard conditions (25° C and 60% relative humidity) or accelerated conditions (40° C and 75% relative humidity).

In another embodiment, the nanoparticulate formulation or pharmaceutical composition exhibits less than 0.5, 1, or 2% total impurities when tested initially and after 3 or 6 months of storage under standard conditions (25° C and 60% relative humidity) or accelerated conditions (40° C and 75% relative humidity). In yet another embodiment, the nanoparticulate formulation or pharmaceutical composition exhibits less than a 3, 5, or 7% variation in the drug content when tested initially and after 3 or 6 months of storage under standard conditions (25° C and 60% relative humidity) or accelerated conditions (40° C and 75% relative humidity).

In an embodiment, the nanoparticulate formulation is in the form of granules that are rapidly dissolvable, for example, dissolving at least 80% of the drug content within 60 minutes, when measured using a USP type II (paddle) apparatus in 900 mL of 0.1 N HCl and 3% to 5% cetyl trimethyl ammonium bromide (CTAB) at 37 ± 0.5° C and a speed of 100 rpm.

In another embodiment, the nanoparticulate formulations are rapidly dissolvable, for example, dissolving at least 80% of the drug content within 60 minutes can also be tested using a USP type II (paddle) apparatus in 900 mL of 0.1 N HCl at 37 ± 0.5° C and a speed of 50 rpm.

### Process of Preparation

The preparation of the nanoparticulate formulation (or pharmaceutical composition containing the nanoparticulate formulation) may include various unit operations such as milling, micronization, mixing, homogenizing, sifting, spraying, solubilizing, dispersing, granulating, lubricating, compressing, coating, and/or filling. These processes may be used for preparing the nanoparticulate formulation and pharmaceutical composition of the present invention. The reduction of the particle size may be achieved using various techniques such as dry or wet milling, micronization, high pressure homogenization, controlled precipitation using an anti-solvent, microfluidization and/or supercritical fluid technology.

One embodiment relates to a process for preparation of a nanoparticulate formulation comprising an mPGES-1 inhibitor (such as compound I or its pharmaceutically acceptable salt) and a surface stabilizer. The process comprises the steps of:
a) mixing the mPGES-1 inhibitor or its pharmaceutically acceptable salts with one or more surface stabilizers, water and optionally other excipients to form an aqueous suspension;
b) reducing the particle size of the aqueous suspension with a bead mill or high pressure wet milling and
c) spray drying of aqueous-suspension.

Yet another embodiment is a process for preparation of a nanoparticulate formulation comprising an mPGES-1 inhibitor (such as compound I or its pharmaceutically acceptable salt) and one or more surface stabilizers. The process comprises the steps of:
a) reducing the particle size of the mPGES-1 inhibitor by bead mill or high pressure wet milling and;
b) mixing the mPGES-1 inhibitor with the surface stabilizer and other excipients
c) spray drying of nano-suspension.

Yet another embodiment is a process for preparation of a nanoparticulate formulation comprising an mPGES-1 inhibitor (such as Compound I or its pharmaceutically acceptable salt) and one or more surface stabilizer. The process comprises the steps of:
1. dissolving polymeric stabilizer (such as copovidone and sodium lauryl sulphate) in water (e.g., purified water);
2. dissolving surfactant (such as poloxamer) in purified water and adding the same to solution of step 1;
3. adding the mPGES-1 inhibitor to the solution of step 2 to form suspension preferably a uniform suspension;
4. milling the suspension of step 3 to obtain the desired particle size;
5. sifting the milled suspension of step 4;
6. spray drying the milled suspension of step 5 to obtain granules; and
7. filling the granules of step 6 in, for example, a triple aluminum laminate pouch or optionally filling in capsules or optionally compressing into tablets.

### Methods of Treatment

The present disclosure also relates to a method of treating pain and/or inflammation or a disease or condition associated with pain and/or inflammation (for example, such a disease or condition which is mediated by mPGES-1) by administering to a subject the nanoparticulate formulation (or pharmaceutical composition containing the nanoparticulate formulation) as described herein.

The present invention also relates to a nanoparticle formulation for the treatment of an inflammation and/or pain in a subject, comprising the compound N-(4-chloro-3-(5-oxo-1-(4-(trifluoromethy))phenyl)-4,5-dihydro-1H-1,2,4-triazo)-3-yl) benzyl) pivalamide ("compound I") or its pharmaceutically acceptable salt and a surface stabilizer, wherein the formulation has an effective average particle size in the range from about 20 nm to about 1000 nm.

In one embodiment, the present invention relates to a nanoparticulate formulation for treating pain and/or inflammation or a disease or condition associated with pain and/or inflammation, comprising an mPGES-1 inhibitor (such as compound I or its pharmaceutically acceptable salt) and a surface stabilizer; where the formulation has an effective average particle size in the range from about 20 nm to about 1000 nm. In one embodiment, the effective average particle size is in the range from about 30 nm to about 900 nm, from about 50 nm to 600 nm, from about 70 nm to about 500 nm, or from about 80 nm to 400 nm.

In further embodiment, the said nanoparticulate formulation can be administered to the subject in need thereof once daily, twice daily, thrice daily or four times a day.

In yet another embodiment, the nanoparticulate formulation comprising compound-1 as mPGES-1 inhibitor can be administered to the subject in need thereof at a dose of the mPGES-1 inhibitor of about 10mg to about 500mg.

### EXAMPLES

### EXAMPLE 1: Nanoparticulate formulation comprising Compound I and a surface stabilizer.

| **Ingredients** | **Quantity (mg)** |
|---|---|
| Compound I | 10 |
| Copovidone (Kollidon VA 64) | 40 |
| Sodium lauryl sulfate | 5 |
| Poloxamer 407 | 20 |
| Lauroyl macrogol-32 glycerides (Gelucire 44/14) | 5 |
| Mannitol | 50 |
| Purified water | q.s. |
| Total weight | 130 |

### Manufacturing process:

1. Kollidon, mannitol, and sodium lauryl sulphate were dissolved in the water while stirring to obtain a clear solution.
2. Poloxamer 407 and Gelucire were dissolved in warm water (50±10°C) and this solution was added to step 1 while stirring to obtain a clear solution.
3. Compound I was added to the solution of step 2 while stirring to form a uniform suspension.
4. The suspension of step 3 was milled using a bead mill to obtain the desired particle size distribution (PSD).
5. The milled suspension of step 4 was sifted through 150# (Pot Sieve).
6. The milled suspension of step 5 was spray dried with the help of a spray dryer to obtain granules.
7. The granules of step 6 were filled in a triple aluminum laminate pouch and the pouch was sealed
8. The pouches of step 7 were packed in a HDPE container along with a canister.
9. The granules can be filled in capsules or can be compressed into tablets.

Particle size data for the granules of Example 1 initially and after 24 hours of storage is provided below.

| **Time** | **Particle size (nm)** | | |
|---|---|---|---|
| | **D₁₀** | **D₅₀** | **D₈₀** |
| 0 (Initial) | 128 | 215 | 356 |
| 24 hours | 135 | 225 | 356 |

The particle size of the compound I was determined in water using a Mastersizer 2000 (Malvern® Instruments Ltd., Malvern, United Kingdom). Three readings were taken for each measurement, and the average size was reported.

### EXAMPLE 2: Pharmaceutical composition comprising the nanoparticulate formulation of Example 1.

| **Ingredients** | **Quantity (mg)** |
|---|---|
| Granules of Example 1 (eq. to 30 mg of compound I) | 390 |
| Hard Gelatin Capsules no 1 | - |
| Total weight | 390 |

| | |
|---|---|
| *based on solid contents | |

### Manufacturing process:

1. Target net fill weight (390 mg) of granules of Example 1 was filled in a hard gelatin capsules No 1.
2. The capsules were packed in HDPE container or blister pack.

The pharmaceutical composition was subjected to accelerated stability studies at a temperature of 40°C±2°C and a relative humidity of 75%±5% for a period of 3 months. The drug assay was performed and active contents were analyzed using HPLC. *In-vitro* dissolution studies were performed using USP type II (Paddle) apparatus in 900 ml 0.1N HCl and 3% cetyl trimethyl ammonium bromide (CTAB) as the Dissolution Medium at a temperature of 37± 0.5 °C and a speed of 100 revolutions per minute (RPM) for a period of 60 minutes. The aliquots taken out at 60 minutes were analyzed for active content by HPLC technique. The HPLC parameters include Inertsil ODS 3V, 150 x 4.6 mm, 5µm column at a flow rate of 1.0 ml/min, detection wavelength of 270 nm, column temperature of 25°C, injection volume of 20µl and run time of 14 minutes. Aqueous orthophosphoric acid buffer (pH 2.5): Acetonitrile in the ratio of 35:65 v/v was used as a mobile phase.

Stability and dissolution data for Example 2:

| **Time** | **% Drug dissolved after 60 minutes** | **% Total impurities (NMT 2%)** | **Drug content (%)** |
|---|---|---|---|
| Initial | 98.4 | 0.25 | 95.6 |
| Real time stability studies on storage at temperature 25°C± 2°C and Relative humidity of 60% ± 5% | | | |
| 3 months | 97.8 | 0.25 | 95.8 |
| Accelerated stability studies on storage at temperature 40°C± 2°C and Relative humidity of 75% ± 5% | | | |
| 3 months | 99.4 | 0.3 | 95.7 |

### EXAMPLE 3: Pharmaceutical composition comprising the nanoparticulate formulation of Example 1.

| **Ingredients** | **Quantity (mg)** |
|---|---|
| Granules of Example 1 (eq. 30 mg of compound I) | 390 |
| Microcrystalline cellulose | 50 |
| Silica, colloidal anhydrous | 5 |
| Sodium stearyl fumarate | 5 |
| Hard Gelatin Capsule No 1 | - |
| Total weight | 450 |

### Manufacturing process:

1. Microcrystalline cellulose was sifted through a 40# sieve and mixed with the granules of Example 1 in a suitable blender.
2. Silica colloidal and sodium stearyl fumarate were sifted through a 40# sieve and were added to the mixture of step 1.
3. The target net fill weight (450 mg) was filled into a suitable capsule.
4. The capsules were packed in HDPE container or blister pack.

Stability and dissolution data for Example 3:

| **Time** | **Amount of drug (%) dissolved in 60 minutes** | **% Total impurities (NMT 2%)** | **Drug content (%)** |
|---|---|---|---|
| Initial | 96.7 | 0.22 | 100.5 |
| Real time stability studies on storage at temperature 25°C± 2°C and Relative humidity of 60% ± 5% | | | |
| 3 months | 100.5 | 0.32 | 102.3 |
| 6 months | 97.7 | 0.27 | 102.5 |
| Accelerated stability studies on storage at temperature 40°C± 2°C and Relative humidity of 75% ± 5% | | | |
| 3 months | 96.4 | 0.3 | 101 |
| 6 months | 96 | 0.35 | 102.8 |

The particle size data for the granules used in the capsules of Example 3 is provided below.

| **Time** | **Particle size (nm)** | |
|---|---|---|
| | **D₁₀** | **D₅₀** |
| 0 (Initial) | 153 | 270 |

### EXAMPLE 4: Nanoparticulate formulation comprising compound I and a surface stabilizer.

| **Ingredients** | **Quantity (mg)** | | | | | |
|---|---|---|---|---|---|---|
| | **4A** | **4B** | **4C** | **4D** | **4E** | **4F** |
| compound I | 10 | 10 | 10 | 10 | 10 | 10 |
| Kollidon VA 64 | 40 | 40 | 40 | 40 | 40 | 40 |
| Poloxamer 407 | 20 | 20 | 20 | 30 | 10 | - |
| Gelucire 44/14 | 5 | - | 5 | 5 | 5 | - |
| Sodium Lauryl Sulphate | 5 | 5 | - | 5 | 5 | |
| Mannitol | 50 | 50 | 50 | 50 | 50 | 50 |
| Vitamin ETPGS | - | - | - | - | - | 10 |
| Purified Water | q.s. | q.s. | q.s. | q.s. | q.s. | q.s. |
| **Total weight** | 130 | 125 | 125 | 140 | 120 | 110 |

The compositions described above were prepared according to the process described in Example 1.

### EXAMPLE 5: Nanoparticulate formulation comprising compound I and a surface stabilizer.

| **Ingredients** | **Quantity (mg)** | | | | | |
|---|---|---|---|---|---|---|
| | **5A** | **5B** | **5C** | **5D** | **5E** | **5F** |
| compound I | 10 | 10 | 10 | 10 | 10 | 10 |
| Hypromellose | 40 | 40 | 40 | | | - |
| Hydroxypropyl cellulose | - | - | - | 40 | 40 | 40 |
| Poloxamer 407 | 20 | 20 | - | 20 | - | 20 |
| Gelucire 44/14 | 5 | - | 5 | - | - | 5 |
| Sodium Lauryl Sulphate | 5 | - | - | .. | 5 | 5 |
| Mannitol | 50 | 50 | 50 | 50 | 50 | 50 |
| Vitamin ETPGS | - | - | - | - | - | - |
| Purified Water | q.s. | q.s. | q.s. | q.s. | q.s. | q.s. |
| **Total weight** | 130 | 130 | 120 | 120 | 105 | 130 |

The compositions were prepared according to the process described in Example 1.

### EXAMPLE 6: Nanoparticulate formulation comprising compound I and a surface stabilizer.

| **Ingredients** | **Quantity (mg)** | | | | | |
|---|---|---|---|---|---|---|
| | **6A** | **6B** | **6C** | **6D** | **6E** | **6F** |
| compound I | 100 | 100 | 100 | 100 | 100 | 100 |
| Mannitol | 50 | 60 | 40 | 30 | 20 | 10 |
| HPMC 3 Cps | 50 | 50 | 50 | 50 | 50 | 50 |
| SLS | 10 | 10 | 10 | 10 | 10 | 10 |
| Vitamin ETPGS | 0 | 0 | 0 | 0 | 0 | 0 |
| Poloxamer 407 | 25 | 25 | 25 | 25 | 25 | 25 |
| Water | qs | qs | qs | qs | qs | qs |
| ***Total*** | **235** | 245 | 225 | 215 | 205 | 195 |

| **Roller compaction** | | | | | | |
|---|---|---|---|---|---|---|
| Spray dried granules | **235** | 245 | 225 | 215 | 205 | 195 |
| MCC (ceolous KG802) | 55 | 55 | 55 | 55 | 55 | 55 |
| Colloidal silicon dioxide | 5 | 5 | 5 | 5 | 5 | 5 |
| ***Total*** | **295** | 305 | 285 | 275 | 265 | 255 |

| **Tablet composition** | | | | | | |
|---|---|---|---|---|---|---|
| Compacted granules | 295 | 305 | 285 | 275 | 265 | 255 |
| MCC (Ceolous KG802) | 172 | 162 | 182 | 192 | 202 | 212 |
| Sodium stearyl fumarate | 5.5 | 5.5 | 5.5 | 5.5 | 5.5 | 5.5 |
| Cross carmellose sodium (Ac di sol) | 27.5 | 27.5 | 27.5 | 27.5 | 27.5 | 27.5 |
| ***Total*** | **500** | 500 | 500 | 500 | 500 | 500 |

### Preparation of suspension

1. HPMC, mannitol and SLS were added to the purified water under continuous stirring until they were dissolved
2. Poloxamer 407 or vitamin E TPGS was added to above solution under stirring until it got dissolved
3. Compound I was added to solution of step 2 and stirred for 45 minutes.

### Milling of suspension

1. The suspension was loaded in bead mill or high pressure wet milling
2. The suspension was milled by using 0.2/0.1 mm zirconium beads till the desired particle size distribution (PSD) was obtained.

### Spray drying of the nanosuspension

1. The suspension was spray dried at product temperature of 45-65°c to obtain a free flowing powder.

### Roller compaction

1. Ceolous KG 802, colloidal silicon dioxide and spray dried granules were mixed and sifted through AST #30.
2. The above granules were then compacted by using a roller compactor and sieved through ASTM #30.

### Lubrication of the compacted granules and compression

1. Compacted granules were mixed with ceolous KG802, SSF, Ac-di-sol blended for 10 minutes and compressed into tablets.

The tablets are optionally film coated.

### EXAMPLE 7: Nanoparticulate formulation comprising compound I and a surface stabilizer.

| **Ingredients** | **Quantity (mg)** | | | | | |
|---|---|---|---|---|---|---|
| | **6A** | **6B** | **6C** | **6D** | **6E** | **6F** |
| Compound I | 100 | 100 | 100 | 100 | 100 | 100 |
| Mannitol | 50 | 50 | 50 | 50 | 50 | 50 |
| HPMC 3 Cps | 50 | 50 | 50 | 50 | 50 | 50 |
| SLS | 25 | 0 | 5 | 15 | 20 | 25 |
| Vitamin ETPGS | 0 | 25 | 25 | 25 | 25 | 25 |
| Poloxamer 407 | 25 | 0 | 0 | 0 | 0 | 0 |
| Water | qs | qs | qs | qs | qs | qs |
| ***Total*** | 250 | 225 | 230 | 240 | 245 | 250 |

| **Roller compaction** | | | | | | |
|---|---|---|---|---|---|---|
| Spray dried granules | 250 | 225 | 230 | 240 | 245 | 250 |
| Microcrystalline cellulose (ceolous KG802) | 55 | 55 | 55 | 55 | 55 | 55 |
| Colloidal silicon dioxide | 5 | 5 | 5 | 5 | 5 | 5 |
| ***Total*** | 310 | 285 | 290 | 300 | 305 | 310 |

| **Tablet composition** | | | | | | |
|---|---|---|---|---|---|---|
| Compacted granules | 310 | 285 | 290 | 300 | 305 | 310 |
| Microcrystalline cellulose (ceolous KG802) | 157 | 182 | 177 | 167 | 162 | 157 |
| Sodium stearyl fumarate | 5.5 | 5.5 | 5.5 | 5.5 | 5.5 | 5.5 |
| Cross carmellose sodium(Ac-di-sol) | 27.5 | 27.5 | 27.5 | 27.5 | 27.5 | 27.5 |
| ***Total*** | 500 | 500 | 500 | 500 | 500 | 500 |

The compositions were prepared according to the process described in Example 6.

### EXAMPLE 8: Determination of particle size of nanoparticulate formulation in a pharmaceutical composition (e.g., tablet or capsule).

The tablet containing the nanoparticulate formulation is crushed to get a powder mass. The powder mass can be further subjected to Hot-stage Optical Microscopy technique as described in Yin et al., Journal of Pharmaceutical Sciences Vol. 94 No. 7, July 2005. Briefly, the powder mass is mounted on the slide, which is heated at a controlled rate (e.g., 10°C/min). The particles remaining at higher temperature are confirmed by DSC and variable-temperature powder X-ray diffraction to be crystalline drug particles.

Alternatively, the particle size of the nanoparticulate formulation in a tablet can also be determined by dispersing the tablet in a suitable solvent in which the excipients are highly soluble as against the nanoparticulate formulation such that the nanoparticulate formulation remains in dispersed form. Further, the particle size of the dispersion can be determined by the methods as described above.

In another method, particle size of Compound I containing granules in the pharmaceutical composition can be determined using a PXRD peak broadening technique, followed by applying the Scherrer equation T = Kλ/ βτ cos θ where τ is the mean particle dimension, K is a constant of 0.9, λ is the X-ray wavelength, and βτ is the peak broadening value due to crystal size reduction, i.e., the full-width-at-half-maximal (FWHM) difference in radian at a certain Bragg angle (θ), between a nanoparticulate dispersion and the micronized excipients. (Dantuluri A. et. al., Sciforum e-conference ECPS 2011 Communication).

Further, different imaging techniques or methodologies can be used to expose the particulate formulation contained in the pharmaceutical compositions (e.g., tablet), wherein *in situ* particle size measurements can be performed. Several methods exist which are able to determine the particle size in a matrix, such as Raman spectroscopy, Transmission Electron Microscopy (TEM), Time of Flight Secondary Ion Mass Spectroscopy (TOF-SIMS), FTIR and NIR microscopy and micro-thermal analysis (µTA).

## Claims

1. A nanoparticulate formulation comprising a compound *N*-(4-chloro-3-(5-oxo-1-(4-(trifluoromethyl)phenyl)-4,5-dihydro-1*H*-1,2,4-triazol-3-yl)benzyl)pivalamide (compound I) or its pharmaceutically acceptable salt and one or more surface stabilizers selected from the group consisting of polymers and surfactants.

2. The formulation according to claim 1, wherein compound I acts as mPGES1 inhibitor.

3. The formulation according to claim 1, wherein said formulation having an effective average particle size in the range from about 20 nm to about 1000 nm.

4. The formulation according to claim 1, wherein surface stabilizer is a polymer selected from one or more of polyvinyl pyrrolidone, copovidone, hydroxypropyl cellulose, hydroxyethyl cellulose, hydroxypropylmethyl cellulose, polyethylene glycol, cellulose derivatives, natural gums and/or combinations thereof.

5. The formulation according to claim 1, wherein the weight ratio of the compound I or its pharmaceutically acceptable salt to the polymer ranges from about 1:0.01 to about 1:100.

6. The formulation according to claim 1, wherein surface stabilizer is a surfactant selected from one or more of poloxamer, polyoxyethylene sorbitan esters, polyethoxylated castor oil, glycerol monostearate, phospholipids, benzalkonium chloride, triethanolamine, sodium lauryl sulfate, docusate sodium, vitamin E TPGS and soya lecithin.

7. The formulation according to claim 1, wherein the weight ratio of the compound I or its pharmaceutically acceptable salt to the surfactant ranges from about 1:0.01 to about 1:100.

8. The formulation according to any one of the preceding claims, wherein the effective average particle size is in the range from about 50 nm to about 600 nm.

9. A nanoparticulate formulation comprising a compound *N*-(4-chloro-3-(5-oxo-1-(4-(trifluoromethyl)phenyl)-4,5-dihydro-1*H*-1,2,4-triazol-3-yl)benzyl)pivalamide ("compound I") or pharmaceutically acceptable salt, and mannitol, sodium lauryl sulphate, hydroxy propyl methyl cellulose, poloxamer or vitamin ETPGS, wherein the formulation has an effective average particle size in the range from about 70 nm to about 500 nm.

10. The nanoparticulate formulation according to any one of the claims 1-9, wherein the formulation is in the form of a dispersion, liquid solution, suspension, semi-solid preparation, granules, powder, tablets or capsules.

11. A pharmaceutical composition comprising the nanoparticulate formulation according to any one of the claims 1-10, and a pharmaceutically acceptable excipient.

12. The pharmaceutical composition according to claim 11, wherein the composition is suitable for oral administration.

13. The pharmaceutical composition according to claim 11 or 12 for use in the treatment of an inflammation and/or pain in a subject.

14. A nanoparticle formulation for use in the treatment of an inflammation and/or pain in a subject, comprising a compound *N*-(4-chloro-3-(5-oxo-1-(4-(trifluoromethyl)phenyl)-4,5-dihydro-1*H-*1,2,4-triazol-3-yl)benzyl)pivalamide ("compound I") or its pharmaceutically acceptable salt and one or more surface stabilizers, wherein said formulation having an effective average particle size in the range from about 20 nm to about 1000 nm.

15. Nanoparticle formulation for use according to claim 14, wherein the said formulation is administered to a subject once daily, twice daily, thrice daily or four times a day.

16. Nanoparticle formulation for use according to claim 14, wherein the said formulation is administered to the subject in the dose from 10 mg to 500 mg.

## Patentansprüche

1. Nanopartikelformulierung, umfassend eine Verbindung *N*-(4-Chlor-3-(5-oxo-1-(4-(trifluormethyl)phenyl-4,5-dihydro-1*H*-1,2,4-triazol-3-yl)benzyl)pivalamid (Verbindung I) oder deren pharmazeutisch akzeptables Salz und einen oder mehrere Oberflächenstabilisatoren, die ausgewählt sind aus der Gruppe bestehend aus Polymeren und Tensiden.

2. Formulierung nach Anspruch 1, wobei die Verbindung I als ein mPGES1-Hemmer fungiert.

3. Formulierung nach Anspruch 1, wobei die Formulierung eine effektive durchschnittliche Partikelgröße im Bereich von etwa 20 nm bis etwa 1000 nm aufweist.

4. Formulierung nach Anspruch 1, wobei der Oberflächenstabilisator ein Polymer ist, das ausgewählt ist aus einem oder mehreren der folgenden: Polyvinylpyrrolidon, Copovidon, Hydroxypropylcellulose, Hydroxyethylcellulose, Hydroxypropylmethylcellulose, Polyethylenglykol, Cellulosederivaten, Naturkautschuk und/oder Kombinationen dieser.

5. Formulierung nach Anspruch 1, wobei das Gewichtsverhältnis der Verbindung I oder ihres pharmazeutisch akzeptablen Salzes zu dem Polymer zwischen etwa 1:0,01 und etwa 1:100 liegt.

6. Formulierung nach Anspruch 1, wobei der Oberflächenstabilisator ein Tensid ist, das ausgewählt ist aus einem oder mehreren der folgenden: Poloxamer, Polyoxyethylensorbitanestern, polyethoxyliertem Kastoröl, Glyzerinmonostearat, Phospholipiden, Benzalkoniumchlorid, Triethanolamin, Natriumlaurylsulfat, Docusat-Natrium, Vitamin E TPGS und Sojalecithin.

7. Formulierung nach Anspruch 1, wobei das Gewichtsverhältnis der Verbindung I oder ihres pharmazeutisch akzeptablen Salzes zu dem Tensid zwischen etwa 1:0,01 und etwa 1:100 liegt.

8. Formulierung nach einem der vorstehenden Ansprüche, wobei die effektive durchschnittliche Partikelgröße im Bereich von etwa 50 nm bis etwa 600 nm liegt.

9. Nanopartikelformulierung, umfassend eine Verbindung *N*-(4-Chlor-3-(5-oxo-1-(4-(trifluormethyl)phenyl-4,5-dihydro-1*H*-1,2,4-triazol-3-yl)benzyl)pivalamid (Verbindung I) oder deren pharmazeutisch akzeptables Salz und Mannitol, Natriumlaurylsulfat, Hydroxypropylmethylcellulose, Poloxamer oder Vitamin ETPGS, wobei die Formulierung eine effektive durchschnittliche Partikelgröße im Bereich von etwa 70 nm bis etwa 500 nm aufweist.

10. Nanopartikelformulierung nach einem der Ansprüche 1 bis 9, wobei die Formulierung die Form einer Dispersion, einer flüssigen Lösung, einer Suspension, eines halbfesten Präparats, von Körnern, eines Pulvers, von Tabletten oder Kapseln aufweist.

11. Pharmazeutische Zusammensetzung, welche die Nanopartikelformulierung nach einem der Ansprüche 1 bis 10 und einen pharmazeutisch akzeptablen Hilfsstoff umfasst.

12. Pharmazeutische Zusammensetzung nach Anspruch 11, wobei sich die Zusammensetzung zur oralen Verabreichung eignet.

13. Pharmazeutische Zusammensetzung nach Anspruch 11 oder 12 zu Verwendung in der Behandlung einer Entzündung und/oder von Schmerzen in einem Subjekt.

14. Nanopartikelformulierung zur Verwendung in der Behandlung einer Entzündung und/oder von Schmerzen in einem Subjekt, umfassend eine Verbindung *N*-(4-Chlor-3-(5-oxo-1-(4-(trifluormethyl)phenyl-4,5-dihydro-1*H*-1,2,4-triazol-3-yl)benzyl)pivalamid (Verbindung I) oder deren pharmazeutisch akzeptables Salz und einen oder mehrere Oberflächenstabilisatoren, wobei die Formulierung eine effektive durchschnittliche Partikelgröße im Bereich von etwa 20 nm bis etwa 1000 nm aufweist.

15. Nanopartikelformulierung zur Verwendung nach Anspruch 14, wobei die Formulierung einem Subjekt einmal täglich, zweimal täglich, dreimal täglich oder viermal täglich verabreicht wird.

16. Nanopartikelformulierung zur Verwendung nach Anspruch 14, wobei die Formulierung in einer Dosis von 10 mg bis 500 mg verabreicht wird.

## Revendications

1. Formulation nanoparticulaire comprenant un composé *N*-(4-chloro-3-(5-oxo-1-(4-(trifluorométhyl)phényl)-4,5-dihydro-1*H*-1,2,4-triazol-3-yl)benzyl)pivalamide (composé I) ou son sel pharmaceutiquement acceptable et un ou plusieurs stabilisants de surface choisis dans le groupe constitué par les polymères et les tensioactifs.

2. Formulation selon la revendication 1, le composé I agissant comme inhibiteur de mPGES1.

3. Formulation selon la revendication 1, ladite formulation ayant une taille particulaire moyenne efficace dans la plage comprise entre environ 20 nm et environ 1 000 nm.

4. Formulation selon la revendication 1, le stabilisant de surface étant un polymère choisi parmi un ou plusieurs parmi la polyvinylpyrrolidone, la copovidone, l'hydroxypropylcellulose, l'hydroxyéthylcellulose, l'hydroxypropylméthylcellulose, le polyéthylèneglycol, les dérivés de cellulose, les gommes naturelles et/ou leurs combinaisons.

5. Formulation selon la revendication 1, le rapport pondéral du composé I ou de son sel pharmaceutiquement acceptable sur le polymère étant compris entre environ 1:0,01 et environ 1:100.

6. Formulation selon la revendication 1, le stabilisant de surface étant un tensioactif choisi parmi un ou plusieurs parmi un poloxamère, des esters de polyoxyéthylène sorbitane, une huile de ricin polyéthoxylée, du monostéarate de glycérol, des phospholipides, du chlorure de benzalkonium, la triéthanolamine, du sulfate de sodium lauryl, du docusate de sodium, la vitamine E TPGS et la lécithine de soja.

7. Formulation selon la revendication 1, le rapport pondéral du composé I ou de son sel pharmaceutiquement acceptable sur le tensioactif étant compris entre environ 1:0,01 et environ 1:100.

8. Formulation selon l'une quelconque des revendications précédentes, la taille particulaire moyenne effective étant comprise entre environ 50 nm et environ 600 nm.

9. Formulation nanoparticulaire comprenant un composé *N*-(4-chloro-3-(5-oxo-1-(4-(trifluorométhyl)phényl)-4,5-dihydro-1*H*-1,2,4-triazol-3-yl)benzyl)pivalamide (« composé I ») ou sel pharmaceutiquement acceptable et
du mannitol, du laurylsulfate de sodium, de l'hydroxy propylméthylcellulose, du poloxamère ou de la vitamine ETPGS, la formulation ayant une taille particulaire moyenne efficace dans la plage comprise entre environ 70 nm et environ 500 nm.

10. Formulation nanoparticulaire selon l'une quelconque des revendications 1 à 9, la formulation étant sous la forme d'une dispersion, d'une solution liquide, d'une suspension, d'une préparation semi-solide, de granulés, de poudre, de comprimés ou de capsules.

11. Composition pharmaceutique comprenant la formulation nanoparticulaire selon l'une quelconque des revendications 1 à 10 et un excipient pharmaceutiquement acceptable.

12. Composition pharmaceutique selon la revendication 11, la composition étant appropriée pour une administration orale.

13. Composition pharmaceutique selon la revendication 11 ou 12 destinée à être utilisée dans le traitement d'une inflammation et/ou d'une douleur chez un sujet.

14. Formulation nanoparticulaire destinée à être utilisée dans le traitement d'une inflammation et/ou d'une douleur chez un sujet, comprenant un composé *N*-(4-chloro-3-(5-oxo-1-(4-(trifluorométhyl)phényle)-4,5-dihydro-1*H*-1,2,4-triazol-3-yl)benzyl)pivalamide (« composé I ») ou son sel pharmaceutiquement acceptable et un ou plusieurs stabilisants de surface, ladite formulation ayant une taille particulaire moyenne efficace dans la plage comprise entre environ 20 nm et environ 1 000 nm.

15. Formulation nanoparticulaire destinée à être utilisée selon la revendication 14, ladite formulation étant administrée à un sujet une fois par jour, deux fois par jour, trois fois par jour ou quatre fois par jour.

16. Formulation nanoparticulaire destinée à être utilisée selon la revendication 14, ladite formulation étant administrée au sujet à une dose comprise entre 10 mg à 500 mg.
